# EUROPÄISCHE PATENTSCHRIFT

(11) **EP 2 812 076 B1**
(45) Veröffentlichungstag und Bekanntmachung des Hinweises auf die Patenterteilung: **31.08.2016**
(21) Anmeldenummer: 13701781.0
(22) Anmeldetag: 30.01.2013
(51) Int. Cl.: A61Q 5/02, A61Q 5/12, A61K 8/73, A61K 8/89, A61K 8/92, A61K 8/891

(54) **KONDITIONIERENDES HAARREINIGUNGSMITTEL**
CONDITIONING HAIR-CLEANING AGENT
AGENT DE LAVAGE CAPILLAIRE CONDITIONNANT

(30) Priorität: 08.02.2012 DE 102012201861
(43) Veröffentlichungstag der Anmeldung: 17.12.2014
(73) Patentinhaber: Henkel AG & Co. KGaA, 40589 Düsseldorf (DE)
(72) Erfinder: SCHRÖDER, Thomas, 22395 Hamburg (DE); HENTRICH, Dirk, 22457 Hamburg (DE)
(86) Internationale Anmeldenummer: PCT/EP2013/051733
(87) Internationale Veröffentlichungsnummer: WO 2013/117464

(56) Entgegenhaltungen:
- EP-A1- 0 468 721
- EP-A1- 1 702 974
- EP-A2- 0 674 898
- EP-A2- 1 977 728
- WO-A1-00/45779
- WO-A1-2009/153280
- DE-A1-102008 034 388
- US-A1- 2006 293 197

## Beschreibung

Die Erfindung liegt auf dem Gebiet der Kosmetik und betrifft Reinigungsmittel, die in einem kosmetisch akzeptablen Träger ein anionisches Tensid, ein kationisches Guar-Polymer, eine Silikonemulsion und ein Wachs enthalten.

Kosmetische Reinigungsmittel, wie beispielsweise Haarshampoos, basieren auf klassischen anionischen, amphoteren, zwitterionischen, nichtionischen und/oder kationischen Tensiden. Aufgrund ihres hervorragenden Reinigungs- und Schaumvermögens werden überwiegend anionische Tenside, gegebenenfalls in der Mischung mit geringen Mengen an Co-Tensiden, eingesetzt.

Ein solches handelsübliches Shampoo reinigt die Haare und beseitigt Talg- und/oder Rückstände von Stylingmitteln sowie andere Verschmutzungen von der Haaroberfläche und der Kopfhaut. Während der Reinigung werden aus den Haaren und der Kopfhaut aber auch Lipide und Proteine entfernt, wodurch insbesondere bei häufiger Reinigung eine Schädigung der Haarstruktur und ein Austrocknen der Kopfhaut auftreten kann.

Um diese Nachteile zu beseitigen, enthält eine Vielzahl kosmetischer Reinigungsmittel zusätzlich Pflegestoffe wie beispielsweise Pflanzenöle, Silikone oder spezielle Polymere.

Auf Ölen-, Fetten und/oder Wachsen basierende Pflegestoffe wirken sich jedoch in vielen Fällen nachteilig auf die Schaumeigenschaften und die Lagerstabilität der Reinigungsmittel aus, weshalb in der Vergangenheit entweder zusätzliche (polymere) Stabilisierungsmittel und/oder höhere Tensidmengen eingesetzt wurden.

In dem Dokument WO 97/35548A1 wurden beispielsweise konditionierende Haarshampoos offenbart, die ein Gemisch aus anionischen und amphoteren Tensiden, einer Silikonemulsion und einem kationischen Polymeren enthalten. Die Shampoos enthalten entweder höhere Tensidmengen oder zusätzliche Stabilisierungsmittel (Carbopole).

Aus ökologischen Gründen sind die Hersteller kosmetischer Reinigungsmittel in den letzten Jahren bestrebt, die Effizienz der Mittel zu steigern, ohne höhere Tensidmengen oder zusätzliche Stabilisierungsmittel zu verwenden (optimalere Nutzung der Ressourcen).

Der vorliegenden Erfindung lag die Aufgabe zugrunde, pflegende Reinigungsmittel mit guten Schaumeigenschaften bereitzustellen.

Auf den Einsatz höherer Tensidmengen und/oder zusätzlicher Stabilisierungsmittel sollte möglichst verzichtet, die Wirksamkeit der Mittel jedoch nicht verringert werden.

Die Reinigungsmittel sollten insbesondere für die schonende Reinigung und Pflege der Haare geeignet sein, und diesen nach der Anwendung eine verbesserte Kämmbarkeit sowie verbesserte optische und haptische Eigenschaften verleihen.

Es wurde gefunden, dass sich Reinigungsmittel auf der Basis anionischer Tenside, spezieller kationischer Polymere, Wachse und Silikone hervorragend dafür eignen. Die entsprechenden Reinigungsmittel pflegen und reinigen das Haar und bilden in Verbindung mit Wasser einen cremigen, feinporigen Schaum.

Ein erster Gegenstand der Erfindung ist daher ein kosmetisches Reinigungsmittel, das in einem kosmetisch akzeptablen Träger
a) 6 bis 12 Gew.-% mindestens ein anionisches Tensid,
b) 0,01 bis 1 Gew.-% mindestens ein kationisches Guar-Polymer,
c) 0,05 bis 2 Gew.-% mindestens eine Silikonemulsion, in der die Silikonteilchen einen mittleren Durchmesser von maximal 600 nm aufweisen, und
d) 0,02 bis 1 Gew.-% mindestens ein Wachs enthält.

Die erfindungsgemäßen Zusammensetzungen enthalten die Komponenten a) bis d) in einem kosmetisch akzeptablen Träger. Dieser ist bevorzugt wässrig oder wässrig-alkoholisch. Bevorzugt enthält der kosmetische Träger mindestens 50 Gew.-%, mehr bevorzugt mindestens 60 Gew.-% und besonders bevorzugt mindestens 70 Gew.-% Wasser.

Weiterhin kann der kosmetische Träger 0,01 bis 50 Gew.-%, bevorzugt 0,05 bis 40 Gew.-% und insbesondere 0,1 bis 30 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.

Bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Zu den geeigneten anionischen Tensiden a), die in den erfindungsgemäßen Mitteln eingesetzt werden können, zählen beispielsweise:
- lineare und verzweigte Fettsäuren mit 8 bis 30 C-Atomen (Seifen),
- Ethercarbonsäuren der Formel R-O-(CH₂CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 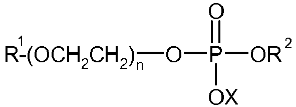 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R⁴R⁵R⁶ steht, mit R³ bis R⁶ unabhängig voneinander stehend für einen C₁ bis C₄ -Kohlenwasserstoffrest.

Bevorzugte anionische Tenside sind Alkylsulfat- und/oder Alkylpolyglykolethersulfatsalze der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet.

Besonders bevorzugte anionische Tenside sind geradkettige oder verzweigte Alkylethersulfate der zuvor genannten Formel, die einen Alkylrest mit 8 bis 18 und insbesondere mit 10 bis 16 C-Atomen sowie 1 bis 6 und insbesondere 2 bis 4 Ethylenoxideinheiten enthalten.

Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Das (oder die) anionische(n) Tensid(e) wird (werden) in den erfindungsgemäßen Reinigungsmitteln - bezogen auf deren Gesamtgewicht - bevorzugt in einer Menge von 6 bis 12 Gew.-%, mehr bevorzugt von 7 bis 11 Gew.-% und insbesondere von 8 bis 10 Gew.-% eingesetzt.

Unter geeigneten kationischen Guar-Polymeren b) sind im Rahmen der vorliegenden Erfindung physiologisch verträgliche kationische Guar-Derivate und/oder hydrophob modifizierte kationische Guar-Derivate zu verstehen.
Bevorzugt sind kationische Hydroxy(C₁-C₄-)alkyl-Guar-Derivate, vorzugsweise kationisches Hydroxyethyltrimethylammonium-Guar und/oder kationisches Hydroxypropyltrimethylammonium-Guar mit mittleren Molekulargewichten (Gewichtsmittel) von 100.000 - 2.000.000 Dalton, bevorzugt von 400.000 - 1.750.000 Dalton und insbesondere von 800.000 -1.600.000 Dalton. Weiterhin bevorzugt sind kationische Hydroxy(C₁-C₄-)alkyl-Guar-Derivate, vorzugsweise kationisches Hydroxyethyltrimethylammonium-Guar und/oder kationisches Hydroxypropyltrimethylammonium-Guar, mit kationischen Ladungsdichten von mindestens 0,5 meq/g.
Insbesondere bevorzugt sind die unter der INCI-Bezeichnung Guar Hydroxypropyltrimonium Chlorid bekannten kationischen Guar-Polymere mit einem Molekulargewicht (Gewichtsmittel) von 100.000 - 2.000.000 Dalton, bevorzugt von 400.000 - 1.750.000 Dalton und insbesondere von 800.000 -1.600.000 Dalton und einer kationischen Ladungsdichte von mindestens 0,5 meq/g.

Geeignete kationische Guar-Polymere b) sind beispielsweise unter den Handelsbezeichnungen "Jaguar^{®}" oder "N-Hance^{®}" von verschiedenen Anbietern erhältlich.
Besonders geeignete kationische Guar-Polymere b) sind: Jaguar^{®} C13S, Jaguar^{®} Excel, N-Hance^{®} 3196 und/oder N-Hance^{®} 3215.

Die kosmetischen Reinigungsmittel enthalten das (oder die) kationische(n) Polymer(e) b) bevorzugt in einer Menge von 0,01 bis 1 Gew.-%, mehr bevorzugt von 0,025 bis 0,75 Gew.-% und insbesondere von 0,05 bis 0,5 Gew.-%, wobei sich die Mengenangaben auf das Gesamtgewicht der Reinigungsmittel beziehen.

Die erfindungsgemäßen Reinigungsmittel enthalten als Konditioniermittel mindestens eine Silikonemulsion, in der die Silikonteilchen einen mittleren Durchmesser von maximal 600 nm aufweisen.
Solche Emulsionen sind im Handel von verschiedenen Anbietern erhältlich.
Ihre Verwendung in den erfindungsgemäßen Reinigungsmitteln weist den Vorteil auf, dass die feinen Silikonteilchen besonders gut auf der Haaroberfläche abgeschieden werden können, ohne dass die Haare dadurch beschwert werden.

Unter dem "mittleren Durchmesser der Silikonteilchen" ist im Sinne der vorliegenden Erfindung bevorzugt der volumenmittlere Silikonteilchendurchmesser D50 zu verstehen, der nach den üblichen Verfahren bestimmt werden kann, beispielsweise durch Laserdiffraktometrie.

Der volumenmittlere Teilchendurchmesser D50 ist derjenige Punkt in der Teilchengrößenverteilung, bei dem 50 Vol.-% der Silikonteilchen einen kleineren und 50 Vol.-% der Silikonteilchen einen größeren Durchmesser aufweisen.

Geeignete Silikone, die in der Silikonemulsionen c) enthalten sein können, können ausgewählt sein aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sein können;
(ii) Polysiloxanen, die in ihrer allgemeinen Struktur eine oder mehrere organofunktionelle Gruppen enthalten, die ausgewählt sein können aus:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Bevorzugte Silikonemulsionen enthalten nichtflüchtige Polyalkylsiloxane, bevorzugt Polydialkylsiloxane, wobei unter bevorzugten Alkylgruppen insbesondere Methylgruppen zu verstehen sind.

Weiterhin bevorzugte Silikonemulsionen enthalten als Silikon Polydimethylsiloxan, welches bei einer Temperatur von 25°C bevorzugt eine Viskosität im Bereich von 1.000 bis 1.000.000 cSt, mehr bevorzugt von 5.000 bis 500.000 cSt, besonders bevorzugt von 10.000 bis 200.000 cSt und insbesondere von 30.000 bis 100.000 cSt aufweist.

Die Viskosität der Polysiloxane kann beispielsweise bei 25°C mit Hilfe eines Glaskapillarviskosimeters nach der Dow Corning Corporate Testmethode CTM0004 (20.07.1970) gemessen werden.

Besonders geeignete Silikonemulsionen sind beispielsweise solche, die bevorzugt
(i) 20 bis 80 Gew.-%, mehr bevorzugt 30 bis 70 Gew.-% und insbesondere 40 bis 60 Gew.-% mindestens eines Polydialkylsiloxans - vorzugsweise eines zuvor definierten Polydimethylsiloxans - sowie
(ii) mindestens zwei unterschiedliche nichtionische Emulgatoren enthalten, die ausgewählt sind aus jeweils einer der beiden Gruppen der 1-5-fach alkoxylierten C₈-C₂₄-Alkohole und der 18-30-fach alkoxylierten C₈-C₂₄-Alkohole,
wobei sich die Mengenangaben auf das Gewicht der Silikonemulsionen beziehen.

Besonders bevorzugte nichtionische Emulgatoren, die in den erfindungsgemäß geeigneten Silikonemulsionen c) eingesetzt werden können, sind ethoxylierte Laurylalkohole. Insbesondere bevorzugt sind die unter den INCI-Bezeichnungen Laureth-4 und Laureth-23 bekannten ethoxylierten Fettalkohole.

Ein Beispiel für eine im Handel erhältliche Silikonemulsion, die bevorzugt in den erfindungsgemäßen Reinigungsmitteln eingesetzt werden kann, ist Xiameter^{®} MEM 1664 (früher: Dow Corning^{®} 1664 Emulsion) von der Firma Dow Corning.
Die Silikonemulsion c) wird in den erfindungsgemäßen Reinigungsmitteln bevorzugt in einer Menge von 0,05 bis 2 Gew.-%, mehr bevorzugt von 0,1 bis 1,5 Gew.-% und insbesondere von 0,2 bis 1 Gew.-% eingesetzt, wobei sich die Mengenangaben auf das Gesamtgewicht der Reinigungsmittel beziehen.

Unter geeigneten "Wachsen" (d)) sind im Rahmen der Erfindung natürliche und synthetische Substanzen zu verstehen, die üblicherweise die folgenden Eigenschaften aufweisen: bei 20°C knetbar, fest bis brüchig, grob bis feinkristallin, durchscheinend bis opak, jedoch nicht glasartig, über 40°C ohne Zersetzung schmelzend, schon wenig oberhalb des Schmelzpunktes verhältnismäßig niedrigviskos und nicht fadenziehend, stark temperaturabhängige Konsistenz und Löslichkeit, unter leichtem Druck polierbar.

Unter natürlichen Wachsen sind vorzugsweise pflanzliche Wachse wie Carnaubawachs, Candelillawachs und/oder Jojobaöl sowie tierische Wachse wie Bienenwachs, Wollwachs, Walrat und/oder Bürzeldrüsenfett zu verstehen.
Unter synthetischen Wachsen sind vorzugsweise Mineralwachse wie Hartparaffin, Ceresin, Ozokerit, Esterwachse wie Polyethylenglycol- oder Polyethylenglycolesterwachse und/oder gehärtete Pflanzenöle zu verstehen.

Bevorzugt enthalten die erfindungsgemäßen Reinigungsmittel mindestens ein chemisch modifiziertes (insbesondere gehärtetes) oder nicht modifiziertes Wachs pflanzlichen Ursprungs, welches bevorzugt einen Schmelzpunkt im Bereich von 80 bis 90°C, mehr bevorzugt von 82 bis 90°C und insbesondere von 85 bis 88°C aufweist.
Besonders bevorzugt enthalten die erfindungsgemäßen Reinigungsmittel mindestens ein gehärtetes Pflanzenöl, insbesondere bevorzugt gehärtetes Rizinusöl.
Das (oder die) Wachs(e) kann (können) in den erfindungsgemäßen Reinigungsmitteln bevorzugt in einer Menge von 0,02 bis 1 Gew.-%, mehr bevorzugt von 0,03 bis 0,75 Gew.-% und insbesondere von 0,05 bis 0,5 Gew.-% enthalten sein, wobei sich die Mengenangaben auf das Gesamtgewicht der Reinigungsmittel beziehen.

In einer ersten bevorzugten Ausführungsform enthalten erfindungsgemäße Reinigungsmittel - jeweils bezogen auf ihr Gesamtgewicht -
a) 6 bis 12 Gew.-%, bevorzugt 7 bis 11 Gew.-% und insbesondere 8 bis 10 Gew.-% mindestens eines anionischen Tensids,
b) 0,01 bis 1 Gew.-%, bevorzugt 0,025 bis 0,75 Gew.-% und insbesondere 0,05 bis 0,5 Gew.-% mindestens eines kationischen Guar-Polymeren,
c) 0,05 bis 2 Gew.-%, bevorzugt 0,1 bis 1,5 Gew.-% und insbesondere 0,2 bis 1 Gew.-% mindestens einer Silikonemulsion, die eine durchschnittliche Silikon-Volumenpartikelgröße von maximal 600 nm aufweist, und
d) 0,02 bis 1 Gew.-%, bevorzugt 0,03 bis 0,75 Gew.-% und insbesondere 0,05 bis 0,5 Gew.-% mindestens eines Wachses.

Innerhalb dieser Ausführungsform ist es besonders bevorzugt, wenn die erfindungsgemäßen Reinigungsmittel -jeweils bezogen auf ihr Gesamtgewicht -
a) 6 bis 12 Gew.-%, bevorzugt 7 bis 11 Gew.-% und insbesondere 8 bis 10 Gew.-% mindestens eines Alkylsulfat- und/oder Alkylpolyglykolethersulfatsalzes der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
b) 0,01 bis 1 Gew.-%, bevorzugt 0,025 bis 0,75 Gew.-% und insbesondere 0,05 bis 0,5 Gew.-% mindestens eines kationischen Hydroxy(C₁-C₄-)alkyl-Guar-Derivates, das ein mittleres Molekulargewicht (Gewichtsmittel) von 100.000 - 2.000.000 Dalton, bevorzugt von 400.000 - 1.750.000 Dalton und insbesondere von 800.000 -1.600.000 Dalton aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist, und
d) 0,02 bis 1 Gew.-%, bevorzugt 0,03 bis 0,75 Gew.-% und insbesondere 0,05 bis 0,5 Gew.-% mindestens eines Wachses pflanzlichen Ursprungs enthalten, welches bevorzugt einen Schmelzpunkt im Bereich von 80 bis 90°C aufweist.

Insbesondere bevorzugte Reinigungsmittel innerhalb dieser ersten bevorzugten Ausführungsform enthalten - bezogen auf das Gesamtgewicht der Reinigungsmittel -
a) 6 bis 12 Gew.-%, bevorzugt 7 bis 11 Gew.-% und insbesondere 8 bis 10 Gew.-% mindestens eines geradkettigen oder verzweigten Alkylethersulfats der zuvor genannten Formel, das einen Alkylrest mit 8 bis 18 und insbesondere mit 10 bis 16 C-Atomen sowie 1 bis 6 und insbesondere 2 bis 4 Ethylenoxideinheiten aufweist,
b) 0,01 bis 1 Gew.-%, bevorzugt 0,025 bis 0,75 Gew.-% und insbesondere 0,05 bis 0,5 Gew.-% mindestens eines unter der INCI-Bezeichnung Guar Hydroxypropyltrimonium Chlorid bekannten kationischen Guar-Polymeren, das ein Molekulargewicht (Gewichtsmittel) von 100.000 - 2.000.000 Dalton, bevorzugt von 400.000 - 1.750.000 Dalton und insbesondere von 800.000 -1.600.000 Dalton und eine kationische Ladungsdichte von mindestens 0,5 meq/g aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist, und
d) 0,02 bis 1 Gew.-%, bevorzugt 0,03 bis 0,75 Gew.-% und insbesondere 0,05 bis 0,5 Gew.-% mindestens eines gehärteten Pflanzenöls, bevorzugt gehärtetes Rizinusöl.

Zur Steigerung der Haarkonditionierenden Eigenschaften können erfindungsgemäße Reinigungsmittel in einer weiteren bevorzugten Ausführungsform zusätzlich 0,01 bis 1 Gew.-%, bevorzugt 0,025 bis 0,75 Gew.-% und insbesondere 0,05 bis 0,5 Gew.-% mindestens eines pflanzlichen Öls enthalten.

Unter geeigneten pflanzlichen (natürlichen, nativen) Ölen sind im Rahmen der vorliegenden Erfindung bevorzugt Triglyceride und Mischungen von Triglyceriden zu verstehen. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter, Shea-Butter und/oder Gemische dieser Öle.

In einer zweiten bevorzugten Ausführungsform enthalten erfindungsgemäße Reinigungsmittel daher -jeweils bezogen auf ihr Gesamtgewicht -
a) 6 bis 12 Gew.-%, bevorzugt 7 bis 11 Gew.-% und insbesondere 8 bis 10 Gew.-% mindestens eines anionischen Tensids,
b) 0,01 bis 1 Gew.-%, bevorzugt 0,025 bis 0,75 Gew.-% und insbesondere 0,05 bis 0,5 Gew.-% mindestens eines kationischen Guar-Polymeren,
c) 0,05 bis 2 Gew.-%, bevorzugt 0,1 bis 1,5 Gew.-% und insbesondere 0,2 bis 1 Gew.-% mindestens einer Silikonemulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines Wachses, und
e) 0,01 bis 1 Gew.-%, bevorzugt 0,025 bis 0,75 Gew.-% und insbesondere 0,05 bis 0,5 Gew.-% mindestens eines pflanzlichen Öls.

Innerhalb dieser Ausführungsform ist es besonders bevorzugt, wenn die erfindungsgemäßen Reinigungsmittel -jeweils bezogen auf ihr Gesamtgewicht -
a) 6 bis 12 Gew.-%, bevorzugt 7 bis 11 Gew.-% und insbesondere 8 bis 10 Gew.-% mindestens eines Alkylsulfat- und/oder Alkylpolyglykolethersulfatsalzes der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
b) 0,01 bis 1 Gew.-%, bevorzugt 0,025 bis 0,75 Gew.-% und insbesondere 0,05 bis 0,5 Gew.-% mindestens eines kationischen Hydroxy(C₁-C₄-)alkyl-Guar-Derivates, das ein mittleres Molekulargewicht (Gewichtsmittel) von 100.000 - 2.000.000 Dalton, bevorzugt von 400.000 - 1.750.000 Dalton und insbesondere von 800.000 -1.600.000 Dalton aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines Wachses pflanzlichen Ursprungs enthalten, welches bevorzugt einen Schmelzpunkt im Bereich von 80 bis 90°C aufweist, und
e) 0,01 bis 1 Gew.-%, bevorzugt 0,025 bis 0,75 Gew.-% und insbesondere 0,05 bis 0,5 Gew.-% mindestens eines pflanzlichen Öls, ausgewählt aus Kokosnussöl, (süßem) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter, Shea-Butter und/oder Gemischen dieser Öle, enthalten.

Insbesondere bevorzugte Reinigungsmittel innerhalb dieser zweiten bevorzugten Ausführungsform enthalten - bezogen auf das Gesamtgewicht der Reinigungsmittel -
a) 6 bis 12 Gew.-%, bevorzugt 7 bis 11 Gew.-% und insbesondere 8 bis 10 Gew.-% mindestens eines geradkettigen oder verzweigten Alkylethersulfats der zuvor genannten Formel, das einen Alkylrest mit 8 bis 18 und insbesondere mit 10 bis 16 C-Atomen sowie 1 bis 6 und insbesondere 2 bis 4 Ethylenoxideinheiten aufweist,
b) 0,01 bis 1 Gew.-%, bevorzugt 0,025 bis 0,75 Gew.-% und insbesondere 0,05 bis 0,5 Gew.-% mindestens eines unter der INCI-Bezeichnung Guar Hydroxypropyltrimonium Chlorid bekannten kationischen Guar-Polymeren, das ein Molekulargewicht (Gewichtsmittel) von 100.000 - 2.000.000 Dalton, bevorzugt von 400.000 - 1.750.000 Dalton und insbesondere von 800.000 -1.600.000 Dalton und eine kationische Ladungsdichte von mindestens 0,5 meq/g aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines gehärteten Pflanzenöls, bevorzugt gehärtetes Rizinusöl, und
e) 0,01 bis 1 Gew.-%, bevorzugt 0,025 bis 0,75 Gew.-% und insbesondere 0,05 bis 0,5 Gew.-% (süßes) Mandelöl, Pfirsichkernöl, Aprikosenkernöl, Avocadoöl, Sojaöl, Sesamöl, Sonnenblumenöl, Traubenkernöl, Amaranthsamenöl, Arganöl, Olivenöl, Jojobaöl und/oder Gemische dieser Öle.

Für einige Anwendungsformen (z.B. für sensitive Reinigungsformulierungen) kann es von Vorteil sein, wenn die erfindungsgemäßen Reinigungsmittel eine besonders milde Tensidbasis enthalten. "Eine besonders milde Tensidbasis" enthalten solche erfindungsgemäßen Reinigungsmittel, die neben dem anionischen Tensid a) bevorzugt 0,1 bis 5 Gew.-%, bevorzugt 0,2 bis 4 Gew.-% und insbesondere 0,3 bis 3 Gew.-% mindestens eines amphoteren, zwitterionischen und/oder nichtionischen Tensids enthalten, wobei sich die Mengenangaben auf das Gesamtgewicht des Reinigungsmittels beziehen.

Geeignete amphotere und/oder zwitterionische Tenside können bevorzugt ausgewählt sein aus einer oder mehreren Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

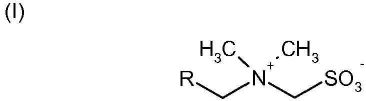

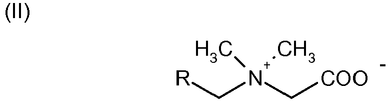

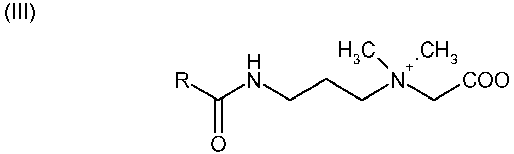

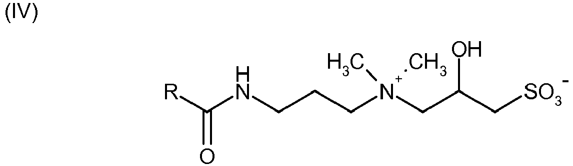

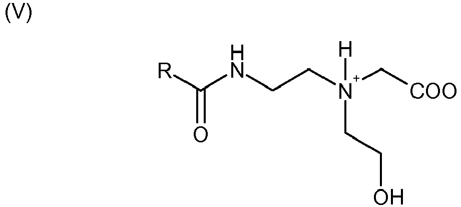

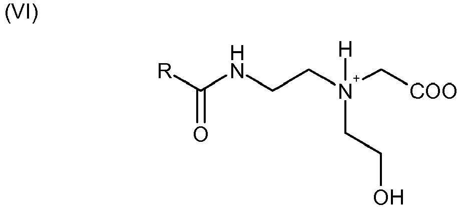

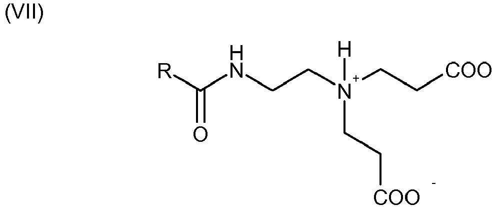

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 16 und insbesondere mit 8 bis 12 C-Atomen.

Mehr bevorzugt sind amphotere und/oder zwitterionische Tenside, bei denen sich der Rest R von Kokosfett ableitet.

Insbesondere bevorzugt sind die unter den INCI-Bezeichnungen Sodium Cocoamphoacetate, Disodium Cocoamphodiacetate, Sodium Cocoamphopropionate, Disodium Cocoamphodipropionate, Coco Betaine, Lauryl Betaine und/oder Cocamidopropylbetain bekannten und im Handel von mehreren Anbietern erhältlichen amphoteren/zwitterionischen Tenside.

Zu den geeigneten nichtionischen Tensiden/Emulgatoren, die in den erfindungsgemäßen Reinigungsmitteln bevorzugt eingesetzt werden können, zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Fettsäurealkanolamide der allgemeinen Formel (VIII), 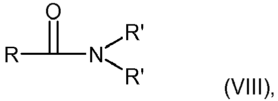 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside.

Besonders bevorzugte nichtionische Tenside, die in den erfindungsgemäßen Reinigungsmitteln eingesetzt werden können, sind Fettsäurealkanolamide der zuvor genannten Formel (VIII). Insbesondere bevorzugt ist ein unter der INCI-Bezeichnung Cocamide MEA bekanntes und im Handel von verschiedenen Anbietern erhältliches Fettsäurealkanolamid.

In einer dritten bevorzugten Ausführungsform enthalten erfindungsgemäße Reinigungsmittel - jeweils bezogen auf ihr Gesamtgewicht -
a) 6 bis 12 Gew.-%, bevorzugt 7 bis 11 Gew.-% und insbesondere 8 bis 10 Gew.-% mindestens eines anionischen Tensids,
b) 0,01 bis 1 Gew.-%, bevorzugt 0,025 bis 0,75 Gew.-% und insbesondere 0,05 bis 0,5 Gew.-% mindestens eines kationischen Guar-Polymeren,
c) 0,05 bis 2 Gew.-%, bevorzugt 0,1 bis 1,5 Gew.-% und insbesondere 0,2 bis 1 Gew.-% mindestens einer Silikonemulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines Wachses, und
e) 0,1 bis 5 Gew.-%, bevorzugt 0,2 bis 4 Gew.-% und insbesondere 0,3 bis 3 Gew.-% mindestens eines amphoteren, zwitterionischen und/oder nichtionischen Tensids.

Innerhalb dieser Ausführungsform ist es besonders bevorzugt, wenn die erfindungsgemäßen Reinigungsmittel -jeweils bezogen auf ihr Gesamtgewicht -
a) 6 bis 12 Gew.-%, bevorzugt 7 bis 11 Gew.-% und insbesondere 8 bis 10 Gew.-% mindestens eines Alkylsulfat- und/oder Alkylpolyglykolethersulfatsalzes der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
b) 0,01 bis 1 Gew.-%, bevorzugt 0,025 bis 0,75 Gew.-% und insbesondere 0,05 bis 0,5 Gew.-% mindestens eines kationischen Hydroxy(C₁-C₄-)alkyl-Guar-Derivates, das ein mittleres Molekulargewicht (Gewichtsmittel) von 100.000 - 2.000.000 Dalton, bevorzugt von 400.000 - 1.750.000 Dalton und insbesondere von 800.000 -1.600.000 Dalton aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines Wachses pflanzlichen Ursprungs enthalten, welches bevorzugt einen Schmelzpunkt im Bereich von 80 bis 90°C aufweist,
e) 0,01 bis 1 Gew.-%, bevorzugt 0,025 bis 0,75 Gew.-% und insbesondere 0,05 bis 0,5 Gew.-% mindestens eines pflanzlichen Öls, und
f) 0,1 bis 5 Gew.-%, bevorzugt 0,2 bis 4 Gew.-% und insbesondere 0,3 bis 3 Gew.-% mindestens eines amphoteren, zwitterionischen und/oder nichtionischen Tensids enthalten.

Insbesondere bevorzugte Reinigungsmittel innerhalb dieser dritten bevorzugten Ausführungsform enthalten - bezogen auf das Gesamtgewicht der Reinigungsmittel -
a) 6 bis 12 Gew.-%, bevorzugt 7 bis 11 Gew.-% und insbesondere 8 bis 10 Gew.-% mindestens eines geradkettigen oder verzweigten Alkylethersulfats der zuvor genannten Formel, das einen Alkylrest mit 8 bis 18 und insbesondere mit 10 bis 16 C-Atomen sowie 1 bis 6 und insbesondere 2 bis 4 Ethylenoxideinheiten aufweist,
b) 0,01 bis 1 Gew.-%, bevorzugt 0,025 bis 0,75 Gew.-% und insbesondere 0,05 bis 0,5 Gew.-% mindestens eines unter der INCI-Bezeichnung Guar Hydroxypropyltrimonium Chlorid bekannten kationischen Guar-Polymeren, das ein Molekulargewicht (Gewichtsmittel) von 100.000 - 2.000.000 Dalton, bevorzugt von 400.000 - 1.750.000 Dalton und insbesondere von 800.000 -1.600.000 Dalton und eine kationische Ladungsdichte von mindestens 0,5 meq/g aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines gehärteten Pflanzenöls, bevorzugt gehärtetes Rizinusöl,
e) 0,01 bis 1 Gew.-%, bevorzugt 0,025 bis 0,75 Gew.-% und insbesondere 0,05 bis 0,5 Gew.-% (süßes) Mandelöl, Pfirsichkernöl, Aprikosenkernöl, Avocadoöl, Sojaöl, Sesamöl, Sonnenblumenöl, Traubenkernöl, Amaranthsamenöl, Arganöl, Olivenöl, Jojobaöl und/oder Gemische dieser Öle, und
f) 0,1 bis 5 Gew.-%, bevorzugt 0,2 bis 4 Gew.-% und insbesondere 0,3 bis 3 Gew.-% mindestens eines der unter den INCI-Bezeichnungen Sodium Cocoamphoacetate, Disodium Cocoamphodiacetate, Sodium Cocoamphopropionate, Disodium Cocoamphodipropionate, Coco Betaine, Lauryl Betaine, Cocamidopropylbetain und/oder Cocamide MEA bekannten Tenside.

Neben den zuvor genannten Inhaltsstoffen können die erfindungsgemäßen Reinigungsmittel noch einer Reihe weiterer fakultativer Wirkstoffe enthalten, die ihnen vorteilhafte Eigenschaften verleihen. Diese können beispielsweise ausgewählt sein aus:
- weiteren - von b) verschiedenen - kationschen Polymeren,
- Vitaminen, Vitaminderivaten und/oder Vitaminvorstufen,
- weiteren - von c) und/oder d) verschiedenen - Ölen, Fetten und/oder Wachsen,
- Proteinhydrolysaten,
- Perlglanzmitteln,
- Pflanzenextrakten und/oder
- Antischuppenmitteln.

Unter besonders geeigneten weiteren - von b) verschiedenen - kationischen Polymeren werden bevorzugt quaternisierte Cellulosederivate verstanden.
Bevorzugte quaternäre Cellulosederivate sind polymere quaternäre Ammoniumsalze, die bei der Umsetzung von Hydroxyethylcellulose mit Trimethylammonium-substituierten Epoxiden entstehen, beispielsweise die unter der INCI-Bezeichnung Polyquaternium-10 bekannten kationischen Polymere.
Polyquaternium-10 ist im Handel von mehreren Anbietern erhältlich.
Für die erfindungsgemäßen Reinigungsmittel geeignet sind beispielsweise die unter den Handelsbezeichnungen Celquat^{®}, Polymer JR^{®} oder Polymer LR^{®} bekannten Polymere. Insbesondere geeignet ist Polymer JR^{®} 400 von der Firma Amerchol.
Das oder die weiteren - von b) verschiedenen - kationische(n) Polymer(e) wird (werden) in den erfindungsgemäßen Reinigungsmitteln - bezogen auf deren Gesamtgewicht - bevorzugt in einer Menge von 0,01 bis 3 Gew.-%, mehr bevorzugt von 0,02 bis 2 Gew.-%, besonders bevorzugt von 0,03 bis 1,5 Gew.-% und insbesondere von 0,05 bis 1 Gew.-% eingesetzt.

Geeignete Vitamine, Vitaminderivate und/oder Vitaminvorstufen können in den erfindungsgemäßen Reinigungsmitteln - bezogen auf das Gesamtgewicht der Reinigungsmittel - bevorzugt in einer Menge von 0,001 bis 5 Gew.-%, mehr bevorzugt von 0,002 bis 4 Gew.-% und besonders bevorzugt von 0,0025 bis 3 Gew.-% enthalten sein.
Unter geeigneten Vitaminen, Vitaminderivaten und Vitaminvorstufen sind bevorzugt Vitamine, Vitaminderivate und Vitaminvorstufen zu verstehen, die den Gruppen A, B, C, E, F und H zugeordnet werden.
*Vitamin A*: Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. *Vitamin B*: Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt.
- Vitamin B₅ (Pantothensäure, Panthenol und Pantolacton). Im Rahmen dieser Gruppe wird bevorzugt das Panthenol und/oder Pantolacton in den erfindungsgemäßen Mitteln eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
- Vitamin B₆ (Pyridoxin sowie Pyridoxamin und Pyridoxal).

*Vitamin C* (Ascorbinsäure): Die Verwendung in Form des Palmitinsäureesters, der Glucoside oder Phosphate kann bevorzugt sein. Die Verwendung in Kombination mit Tocopherolen kann ebenfalls bevorzugt sein.
*Vitamin E* (Tocopherole, insbesondere α-Tocopherol): Darunter fallen Tocopherol und seine Derivate, worunter insbesondere die Ester wie das Acetat, das Nicotinat, das Phosphat und das Succinat zu verstehen sind.
*Vitamin F*: Unter dem Begriff "Vitamin F" werden üblicherweise essentielle Fettsäuren, insbesondere Linolsäure, Linolensäure und Arachidonsäure, verstanden.
*Vitamin H:* Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat.
Erfindungsgemäß bevorzugte Reinigungsmittel enthalten mindestens ein Vitamin, Vitaminderivat oder eine Vitaminvorstufe aus den zuvor genannten Gruppen A, B, E und H.
Besonders bevorzugte erfindungsgemäße Reinigungsmittel enthalten mindestens ein Vitamin, Vitaminderivat oder eine Vitaminvorstufe der B-Gruppe.
Insbesondere bevorzugte erfindungemäße Reinigungsmittel enthalten Niacinamid, Panthenol, Pantolacton und/oder Pyridoxin.

Weitere geeignete - von c) und/oder d) verschiedene - Öl-, Fett- und/oder Wachskomponenten können in den erfindungsgemäßen Reinigungsmitteln bevorzugt in einer Menge von 0,001 bis 10 Gew.-%, mehr bevorzugt von 0,005 bis 7,5 Gew.-% und insbesondere von 0,01 bis 5 Gew.-% eingesetzt werden, wobei sich die Mengenangaben auf das Gesamtgewicht des finalen Reinigungsmittels beziehen.
Sie können ausgewählt sein aus natürlichen, synthetischen und/oder mineralischen Öl-, Fett- und/oder Wachskomponenten.
Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).
Als Ölkomponente kann weiterhin ein Dialkylether dienen.
Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.
Besonders bevorzugt ist der Di-n-octylether, der im Handel unter der Bezeichnung Cetiol^{®} OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können.
Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.
Als weitere - von d) verschiedene - natürliche oder synthetische Wachse können eingesetzt werden Sonnenblumenwachs, Fruchtwachse, wie beispielsweise Apfelwachs oder Citruswachs, und/oder Microwachse aus PE- oder PP. Derartige Wachse sind beispielsweise erhältlich über die Fa. Kahl & Co., Trittau.
Weitere Fettstoffe sind beispielsweise
- Esteröle. Unter Esterölen sind zu verstehen die Ester von C₆ - C₃₀ - Fettsäuren mit C₂ - C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Geeignete Proteinhydrolysate, die in den erfindungsgemäßen Reinigungsmitteln eingesetzt werden können, sind vorzugsweise pflanzlichen, tierischen oder marinen Ursprungs. Sie können in den erfindungsgemäßen Mitteln bevorzugt in einer Menge von 0,01 bis 10 Gew.-%, mehr bevorzugt von 0,25 bis 7,5 Gew.-% und insbesondere von 0,05 bis 5 Gew.-% eingesetzt werden, wobei sich die Mengenangaben auf das Gesamtgewicht des finalen Reinigungsmittels beziehen.
Geeignete tierische Proteinhydrolysate sind beispielsweise Elastin-, Kollagen-, Keratin-, Seiden- und/oder Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{Ⓡ} (Deutsche Gelatine Fabriken Stoess & Co), Lexein^{®} (Inolex) und Kerasol^{Ⓡ} (Croda) vertrieben.

Geeignete Proteinhydrolysate pflanzlichen Ursprungs sind beispielsweise Soja-, Mandel-, Reis-, Erbsen-, Kartoffel-, Raps- und/oder Weizenproteinhydrolysate.
Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.
Zu den geeigneten Proteinhydrolysaten marinen Ursprunges zählen beispielsweise Kollagenhydrolysate von Fischen oder Algen sowie Proteinhydrolysate von Muscheln bzw. Perlenhydrolysate. Beispiele für geeignete Perlenhydrolysate sind die Handelsprodukte Pearl Protein Extract BG^{®} oder Crodarom^{®} Pearl.
Einsetzbar sind auch kationisierte Proteinhydrolysate, wobei das zugrunde liegende Proteinhydrolysat aus den zuvor beschriebenen tierischen, pflanzlichen und/oder marinen Quellen stammen kann.
Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternärer Ammoniumsalze wie beispielsweise N,N-Dimethyl-N-(n-Alkyl)-N-(2-hydroxy-3-chloro-n-propyl)-ammoniumhalogeniden durchgeführt.
Weiterhin können die kationischen Proteinhydrolysate auch noch weiter derivatisiert sein.
Als typische Beispiele für geeignete kationische Proteinhydrolysate und/oder -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Erfindungsgemäß geeignete Perlglanzmittel sind beispielsweise
- Glycoldistearinsäureester,
- C₈-C₃₀-Fettsäuremonoglycolester und/oder
- mit Titandioxid überzogene Glimmerpigmente,
wie sie beispielsweise unter den Handelsbezeichnungen Rewopal^{®}, Genapol^{®} PMS, Cutina^{®} EGMS, Timiron^{®}, Colorona^{®} und Euperlan^{®} erhältlich sind.
Das (oder die) Perlglanzmittel kann (können) in den erfindungsgemäßen Reinigungsmitteln - bezogen auf deren Gewicht - bevorzugt in Mengen von 0,01 bis 3 Gew.-%, mehr bevorzugt von 0,025 bis 2 Gew.-% und besonders bevorzugt von 0,05 bis 1 Gew.-% eingesetzt werden.

Unter geeigneten Pflanzenextrakten sind Extrakte zu verstehen, die aus allen Teilen einer Pflanze hergestellt werden können.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens bevorzugt.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Der (die) Pflanzenextrakt(e) kann (können) in den erfindungsgemäßen Reinigungsmitteln bevorzugt in einer Menge von 0,001 bis 5 Gew.-%, mehr bevorzugt von 0,002bis 3 Gew.-% und insbesondere von 0,005 bis 2 Gew.-% eingesetzt werden, wobei sich die Mengenangaben auf das Gesamtgewicht der kosmetischen Reinigungsmittel beziehen.

Antischuppenwirkstoffe können in den erfindungsgemäßen kosmetischen Reinigungsmitteln (bezogen auf das Gesamtgewicht der Reinigungsmittel) bevorzugt in einer Menge von 0,025 bis 7,5 Gew.-%, besonders bevorzugt von 0,05 bis 5 Gew.-% und insbesondere von 0,075 bis 3 Gew.-% eingesetzt werden.
Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.
Bevorzugt sind Climbazol, Zink Pyrithion und Piroctone Olamine, insbesondere bevorzugt ist Zink Pyrithion.

Bevorzugte Ausführungsformen der erfindungsgemäßen kosmetischen Reinigungsmittel sind Haarshampoos, Duschbäder, Duschgele, Haarspülungen, Haarkuren, Rasierwässer und/oder Deodorantien. Insbesondere bevorzugt sind erfindungsgemäße Reinigungsmittel, die der Haar- und Kopfhautreinigung dienen.

Erfindungsgemäße Reinigungsmittel weisen bevorzugt einen pH-Wert im Bereich von 4 bis 5,7, mehr bevorzugt von 4,2 bis 5,5 und insbesondere von 4,5 bis 5,3 auf.

Neben den zwingenden und bevorzugten Komponenten können die erfindungsgemäßen Reinigungszubereitungen weitere, dem Fachmann für solche kosmetischen Mittel bekannte Komponenten enthalten.
Unter diese fallen beispielsweise:
- Strukturanten wie Maleinsäure und Milchsäure,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- weitere Substanzen zur Einstellung des pH-Wertes, wie beispielsweise α- und β-Hydroxycarbonsäuren,
- Wirkstoffe wie Allantoin und Bisabolol,
- Komplexbildner wie EDTA, NTA, β-Alanindiessigsäure und Phosphonsäuren,
- Treibmittel wie Propan-Butan-Gemische, N₂O, Dimethylether, CO₂ und Luft,
- Konsistenzgeber wie Zuckerester, Polyolester oder Polyolalkylether,
- Konservierungsmittel, wie beispielsweise Natriumbenzoat oder Salicylsäure,
- Viskositätsregler wie Salze (NaCl).
Die erfindungsgemäßen Reinigungsmittel weisen hervorragende Eigenschaften auf.

Sie sind stabil und bilden in Verbindung mit Wasser einen reichhaltigen Schaum, der sich leicht auf der Anwendungsoberfläche verteilen lässt.
Sie enthalten geringere Mengen verschiedener Pflegekomponenten (kationische Polymere, Silikone, Öle) und/oder geringere Tensidmengen als handelsübliche Shampoos, und weisen dennoch eine vergleichbare Pflegeleistung auf. Die Ressourcen werden bei der Herstellung der erfindungsgemäßen Reinigungsmittel demnach effizienter genutzt.
Mit den erfindungsgemäßen Mitteln behandelte Haare weisen insbesondere eine verbesserte Kämmbarkeit, einen verbesserten Griff und einen erhöhten Glanz auf.

### Beispiele:

Die folgende Tabelle enthält Beispiele für erfindungsgemäße Reinigungszusammensetzungen (Beispiel 2) sowie für Vergleichszusammensetzungen (Beispiele 1 und 3). Die Mengenangaben in der Tabelle beziehen sich - sofern nicht anders angegeben - auf Gew.-%.

| | **1** | **2** | **3** |
|---|---|---|---|
| Natriumlaurethsulfat (2EO) | 10,80 | 9,00 | 7,00 |
| Natriumlaurylsulfat | | | 5,00 |
| Disodium Cocoamphodiacetate | 3,20 | 0,80 | |
| Cocamidopropylbetain | | 1,60 | |
| Ammonium Xylenesulfonate | | | 1,00 |
| Cocamide MEA | | 0,45 | 0,60 |
| PEG-7 Glyceryl Cocoate | 1,20 | 0,40 | 0,40 |
| PEG-40 Hydrogenated Castor Oil | | 0,30 | 0,30 |
| PEG-55 Propylene Glycol Oleate | 0,40 | | |
| Laureth-2 | 0,40 | | |
| Polymer JR^{Ⓡ1} 400 | 0,70 | | 0,30 |
| Guar Hydroxypropyl Trimonium Chloride | | 0,30 | |
| PEG-12 Dimethicone | 1,00 | | |
| Xiameter^{Ⓡ2} MEM 1664 Emulsion | | 0,40 | 1,20 |
| Mandelöl, süß | | 0,05 | |
| Hydrogenated Castor Oil | 0,20 | 0,10 | 0,10 |
| Perlglanzmittel | 0,10 | 0,10 | 0,10 |
| Elektrolyt (z.B. NaCl zur Einstellung einer Viskosität von 8,000 bis 9,000 mPas*) | 0,60 | 1,20 | 1,10 |
| Säuerungsmittel, Konservierungsmittel, Parfum | q.s. | q.s. | q.s. |
| Wasser | ad 100 | ad 100 | ad 100 |
| Gesamt-Wirkstoffgehalt (Tenside und Öle/Wachse) | 17,90 | 13,40 | 17,10 |
| Durchschnittliche Bewertung | 5,8 | 5,9 | 5,9 |
| Reduktion der Nasskämmbarkeit | 62 | 65 | 54 |
| Reduktion der Trockenkämmbarkeit | -28 | 51 | 25 |

| | | | |
|---|---|---|---|
| * Die jeweilige Viskosität wurde bestimmt mit einem Haake Rotationsviskosimeter VT550 bei einer Temperatur von 20°C; Messeinrichtung MV; Spindel MV II; 8 UPM. | | | |

In den zuvor genannten Beispielen wurden die folgenden Handelsprodukte eingesetzt:
1 INCI-Bezeichnung: Polyquaternium-10; Dow
2 INCI-Bezeichnung: Dimethicone, Laureth-4, Laureth-23; Dow Corning

Die zuvor genannten Shampoos wurden wie folgt bewertet:
Etwa 200 unabhängige Testpersonen im Alter von 20 bis 60 Jahren (50% im Alter von 20 - 40 Jahren und 50% im Alter von 41 - 60 Jahren) verwendeten die codierten Shampoos in einem Blindtest ("Interviewer-assisted Home Use Test").

Anschließend bewerteten sie die Shampoos auf einer Bewertungsskala von 1 bis 7, wobei "7" sehr zufrieden, "6" zufrieden, "5" kann man verwenden, "4" noch in Ordnung, "3" nicht so gut "2" schlecht und "1" sehr schlecht bedeutet.

Die Bestimmung der Reduktion der Nass- und Trockenkämmbarkeit erfolgte nach der üblichen Methode (WO 2004/26270A1 indem die Reduktion der Kämmkraft einer mit dem jeweiligen Shampoo gewaschenen, blondierten Haarsträhne (nass und trocken!) gegenüber einer mit dem jeweiligen Shampoo gewaschenen, unbehandelten Haarsträhne (nass und trocken!) verglichen wurde.

## Patentansprüche

1. Kosmetisches Reinigungsmittel, enthaltend in einem kosmetisch akzeptablen Träger- bezogen auf sein Gesamtgewicht -
a) 6 bis 12 Gew.-% mindestens eines anionischen Tensids,
b) 0,01 bis 1 Gew.-% mindestens eines kationischen Guar-Polymers,
c) 0,05 bis 2 Gew.-% mindestens einer Silikonemulsion, in der die Silikonteilchen einen mittleren Durchmesser von maximal 600 nm aufweisen, und
d) 0,02 bis 1 Gew.-% mindestens eines Wachses.

2. Kosmetisches Reinigungsmittel nach Anspruch 1, **dadurch gekennzeichnet, dass** es mindestens ein anionisches Tensid, ausgewählt aus Alkylsulfat- und/oder Alkylpolyglykolethersulfatsalzen der Formel R-(OCH₂-CH₂)ₓ-OSO₃ X enthält, in der R bevorzugt für eine lineare oder verzweigte, gesättigte oder ungesättigte Alkylgruppe mit 8 bis 30 C-Atomen, x für 0 oder eine Zahl von 1 bis 12 und X für ein Alkali-, ein Erdalkali-, ein Ammonium- oder ein Alkanolaminion steht.

3. Kosmetisches Reinigungsmittel nach einem der Ansprüche 1 oder 2, **dadurch gekennzeichnet, dass** das kationische Polymer b) ausgewählt ist aus kationischen Hydroxy(C₁-C₄-)alkyl-Guar-Derivaten, vorzugsweise aus kationischem Hydroxyethyltrimethylammonium-Guar und/oder kationischem Hydroxypropyltrimethylammonium-Guar mit mittleren Molekulargewichten (Gewichtsmittel) von 100.000 - 2.000.000 Dalton und kationischen Ladungsdichten von mindestens 0,5 meq/g.

4. Kosmetisches Reinigungsmittel nach einem der Ansprüche 1 bis 3, **dadurch gekennzeichnet, dass** die Silikonemulsion c)
(i) 20 bis 80 Gew.-%, bevorzugt 30 bis 70 Gew.-% und insbesondere 40 bis 60 Gew.-% mindestens eines Polydialkylsiloxans - vorzugsweise eines Polydimethylsiloxans - sowie
(ii) mindestens zwei unterschiedliche nichtionische Emulgatoren enthält, die ausgewählt sind aus jeweils einer der beiden Gruppen der 1-5-fach alkoxylierten C₈-C₂₄-Alkohole und der 18-30-fach alkoxylierten C₈-C₂₄-Alkohole,
wobei sich die Mengenangaben auf das Gewicht der Silikonemulsion c) beziehen.

5. Kosmetisches Reinigungsmittel nach Anspruch 4, **dadurch gekennzeichnet, dass** das Polydimethylsiloxan bei 25°C eine Viskosität im Bereich von 1.000 bis 1.000.000 cSt, bevorzugt von 5.000 bis 500.000 cSt, besonders bevorzugt von 10.000 bis 200.000 cSt und insbesondere von 30.000 bis 100.000 cSt aufweist.

6. Kosmetisches Reinigungsmittel nach einem der Ansprüche 1 bis 5, **dadurch gekennzeichnet, dass** es ein Wachs d) pflanzlichen Ursprungs enthält, welches einen Schmelzpunkt im Bereich von 80 bis 90°C aufweist.

7. Kosmetisches Reinigungsmittel nach Anspruch 6, **dadurch gekennzeichnet, dass** es ein gehärtetes Pflanzenöl - vorzugsweise gehärtetes Rizinusöl - enthält.

8. Kosmetisches Reinigungsmittel nach einem der Ansprüche 1 bis 7, **dadurch gekennzeichnet, dass** es - bezogen auf sein Gesamtgewicht -
a) 7 bis 11 Gew.-% und insbesondere 8 bis 10 Gew.-% mindestens eines anionischen Tensids,
b) 0,025 bis 0,75 Gew.-% und insbesondere 0,05 bis 0,5 Gew.-% mindestens eines kationischen Guar-Polymeren,
c) 0,1 bis 1,5 Gew.-% und insbesondere 0,2 bis 1 Gew.-% mindestens einer Silikonemulsion, die eine durchschnittliche Silikon-Volumenpartikelgröße von maximal 600 nm aufweist, und
d) 0,03 bis 0,75 Gew.-% und insbesondere 0,05 bis 0,5 Gew.-% mindestens eines Wachses enthält.

9. Kosmetisches Reinigungsmittel nach einem der Ansprüche 1 bis 8, **dadurch gekennzeichnet, dass** es - bezogen auf sein Gesamtgewicht - weiterhin 0,01 bis 1 Gew.-%, bevorzugt 0,025 bis 0,75 Gew.-% und insbesondere 0,05 bis 0,5 Gew.-% mindestens eines pflanzlichen Öls enthält.

10. Kosmetisches Reinigungsmittel nach einem der Ansprüche 1 bis 9, **dadurch gekennzeichnet, dass** es - bezogen auf sein Gesamtgewicht -
a) 6 bis 12 Gew.-%, bevorzugt 7 bis 11 Gew.-% und insbesondere 8 bis 10 Gew.-% mindestens eines geradkettigen oder verzweigten Alkylethersulfats der Formel R-(OCH₂-CH₂)ₓ-OSO₃ X enthält, das einen Alkylrest (R) mit 8 bis 18 und insbesondere mit 10 bis 16 C-Atomen sowie 1 bis 6 und insbesondere 2 bis 4 Ethylenoxideinheiten (x) aufweist,
b) 0,01 bis 1 Gew.-%, bevorzugt 0,025 bis 0,75 Gew.-% und insbesondere 0,05 bis 0,5 Gew.-% mindestens eines unter der INCI-Bezeichnung Guar Hydroxypropyltrimonium Chlorid bekannten kationischen Guar-Polymeren, das ein Molekulargewicht (Gewichtsmittel) von 100.000 - 2.000.000 Dalton, bevorzugt von 400.000 - 1.750.000 Dalton und insbesondere von 800.000 -1.600.000 Dalton und eine kationische Ladungsdichte von mindestens 0,5 meq/g aufweist,
c) 0,05 bis 2 Gew.-%, bevorzugt 0,1 bis 1,5 Gew.-% und insbesondere 0,2 bis 1 Gew.-% mindestens einer Dimethylpolysiloxan-Emulsion, die eine durchschnittliche Silikon-Volumenpartikelgröße von maximal 600 nm aufweist,
d) 0,02 bis 1 Gew.-%, bevorzugt 0,03 bis 0,75 Gew.-% und insbesondere 0,05 bis 0,5 Gew.-% mindestens eines gehärteten Pflanzenöls, bevorzugt gehärtetes Rizinusöl, und
e) 0,01 bis 1 Gew.-%, bevorzugt 0,025 bis 0,75 Gew.-% und insbesondere 0,05 bis 0,5 Gew.-% (süßes) Mandelöl, Pfirsichkernöl, Aprikosenkernöl, Avocadoöl, Sojaöl, Sesamöl, Sonnenblumenöl, Traubenkernöl, Amaranthsamenöl, Arganöl, Olivenöl, Jojobaöl und/oder Gemische dieser Öle enthält.

11. Kosmetisches Reinigungsmittel nach einem der Ansprüche 1 bis 10, **dadurch gekennzeichnet, dass** es - bezogen aus sein Gesamtgewicht - weiterhin 0,1 bis 5 Gew.-%, bevorzugt 0,2 bis 4 Gew.-% und insbesondere 0,3 bis 3 Gew.-% mindestens eines amphoteren, zwitterionischen und/oder nichtionischen Tensids enthält.

12. Kosmetisches Reinigungsmittel nach Anspruch 9, **dadurch gekennzeichnet, dass** es mindestens eines der unter den INCI-Bezeichnungen Sodium Cocoamphoacetate, Disodium Cocoamphodiacetate, Sodium Cocoamphopropionate, Disodium Cocoamphodipropionate, Coco Betaine, Lauryl Betaine, Cocamidopropylbetain und/oder Cocamide MEA bekannten Tenside enthält.

13. Kosmetisches Reinigungsmittel nach einem der Ansprüche 1 bis 12, **dadurch gekennzeichnet, dass** es - bezogen aus sein Gesamtgewicht - weiterhin 0,025 bis 7,5 Gew.-%, bevorzugt 0,05 bis 5 Gew.-% und insbesondere 0,075 bis 3 Gew.-% mindestens eines Antischuppenwirkstoffs enthält.

14. Kosmetisches Reinigungsmittel nach einem der Ansprüche 1 bis 13, **dadurch gekennzeichnet, dass** es einen pH-Wert im Bereich von 4 bis 5,7, bevorzugt von 4,2 bis 5,5 und insbesondere von 4,5 bis 5,3 aufweist.

15. Verwendung eines kosmetischen Reinigungsmittels nach einem der Ansprüche 1 bis 14 als Haarshampoo, Duschbad, Duschgel, Haarspülung, Haarkur, Rasierwasser und/oder Deodorant, bevorzugt als Reinigungsmittel, das der Haar- und Kopfhautreinigung dient.

## Claims

1. A cosmetic cleaning agent, containing in a cosmetically acceptable vehicle, based on its total weight:
a) 6 to 12 wt.% of at least one anionic surfactant,
b) 0.01 to 1 wt.% of at least one cationic guar polymer,
c) 0.05 to 2 wt.% of at least one silicone emulsion, in which the silicone particles have an average diameter of at most 600 nm, and
d) 0.02 to 1 wt.% of at least one wax.

2. The cosmetic cleaning agent according to claim 1, **characterized in that** it contains at least one anionic surfactant, selected from alkyl sulfate salts and/or alkyl polyglycol ether sulfate salts of the formula R-(OCH₂-CH₂)ₓ-OSO₃ X, in which R preferably represents a linear or branched, saturated or unsaturated alkyl group having 8 to 30 C atoms, x represents 0 or a number from 1 to 12 and X represents an alkali ion, an earth alkali ion, an ammonium ion or an alkanolamine ion.

3. The cosmetic cleaning agent according to one of claims 1 or 2, **characterized in that** the cationic polymer b) is selected from cationic hydroxy(C₁-C₄)alkyl guar derivatives, preferably from cationic hydroxyethyl trimethylammonium guar and/or cationic hydroxypropyl trimethylammonium guar having average molecular weights (weight average) of 100,000-2,000,000 Dalton and cationic charge densities of at least 0.5 meq/g.

4. The cosmetic cleaning agent according to one of claims 1 to 3, **characterized in that** the silicone emulsion c) contains
(i) 20 to 80 wt.%, preferably 30 to 70 wt.% and in particular 40 to 60 wt.% of at least one polydialkylsiloxane, preferably one polydimethylsiloxane, and
(ii) at least two different non-ionic emulsifiers, which are each selected from one of the two groups of the 1-5 times alkoxylated C₈-C₂₄ alcohols and the 18-30 times alkoxylated C₈-C₂₄ alcohols,
the stated quantities relating to the weight of the silicone emulsion c).

5. The cosmetic cleaning agent according to claim 4, **characterized in that** at 25°C the polydimethylsiloxane has a viscosity in the range between 1,000 and 1,000,000 cSt, preferably 5,000 and 500,000 cSt, particularly preferably 10,000 and 200,000 cSt and in particular 30,000 and 100,000 cSt.

6. The cosmetic cleaning agent according to one of claims 1 to 5, **characterized in that** it comprises a wax d) of plant origin which has a melting point in the range between 80 and 90°C.

7. The cosmetic cleaning agent according to claim 6, **characterized in that** it contains a hydrogenated vegetable oil, preferably hydrogenated castor oil.

8. The cosmetic cleaning agent according to one of claims 1 to 7, **characterized in that** it contains, based on its total weight:
a) 7 to 11 wt.% and in particular 8 to 10 wt.% of at least one anionic surfactant,
b) 0.025 to 0.75 wt.% and in particular 0.05 to 0.5 wt.% of at least one cationic guar polymer,
c) 0.1 to 1.5 wt.% and in particular 0.2 to 1 wt.% of at least one silicone emulsion, which has an average silicone volume particle size of at most 600 nm, and
d) 0.03 to 0.75 wt.% and in particular 0.05 to 0.5 wt.% of at least one wax.

9. The cosmetic cleaning agent according to one of claims 1 to 8, **characterized in that** it also contains, based on its total weight, 0.01 to 1 wt.%, preferably 0.025 to 0.75 wt.% and in particular 0.05 to 0.5 wt.% of at least one vegetable oil.

10. The cosmetic cleaning agent according to one of claims 1 to 9, **characterized in that** it contains, based on its total weight:
a) 6 to 12 wt.%, preferably 7 to 11 wt.% and in particular 8 to 10 wt.% of at least one straight-chain or branched alkyl ether sulfate of the formula R-(OCH₂-CH₂)ₓ-OSO₃ X, which has an alkyl functional group (R) having 8 to 18 and in particular having 10 to 16 C atoms and 1 to 6 and in particular 2 to 4 ethylene oxide units (x),
b) 0.01 to 1 wt.%, preferably 0.025 to 0.75 wt.% and in particular 0.05 to 0.5 wt.% of at least one cationic guar polymer, known under the INCI name guar hydroxypropyltrimonium chloride, which has a molecular weight (average weight) of 100,000 to 2,000,000 Dalton, preferably 400,000 to 1,750,000 Dalton and in particular 800,000 to 1,600,000 Dalton and a cationic charge density of at least 0.5 meq/g,
c) 0.05 to 2 wt.%, preferably 0.1 to 1.5 wt.% and in particular 0.2 to 1 wt.% of at least one dimethylpolysiloxane emulsion, which has an average silicone volume particle size of at most 600 nm,
d) 0.02 to 1 wt.%, preferably 0.03 to 0.75 wt.% and in particular 0.05 to 0.5 wt.% of at least one hydrogenated vegetable oil, preferably hydrogenated castor oil, and
e) 0.01 to 1 wt.%, preferably 0.025 to 0.75 wt.% and in particular 0.05 to 0.5 wt.% (sweet) almond oil, peach kernel oil, apricot seed oil, avocado oil, soybean oil, sesame oil, sunflower oil, grape seed oil, amaranth seed oil, argan oil, olive oil, jojoba oil and/or mixtures of these oils.

11. The cosmetic cleaning agent according to one of claims 1 to 10, **characterized in that** it also contains, based on its total weight, 0.1 to 5 wt.%, preferably 0.2 to 4 wt.% and in particular 0.3 to 3 wt.% of at least one amphoteric, zwitterionic and/or non-ionic surfactant.

12. The cosmetic cleaning agent according to claim 9, **characterized in that** it contains at least one of the surfactants known under the INCI names sodium cocoamphoacetate, disodium cocoamphodiacetate, sodium cocoamphopropionate, disodium cocoamphodipropionate, coco betaine, lauryl betaine, cocamidopropylbetain and/or cocamide MEA.

13. The cosmetic cleaning agent according to one of claims 1 to 12, **characterized in that** it also contains, based on its total weight, 0.025 to 7.5 wt.%, preferably 0.05 to 5 wt.% and in particular 0.075 to 3 wt.% of at least one anti-dandruff active ingredient.

14. The cosmetic cleaning agent according to one of claims 1 to 13, **characterized in that** it has a pH in the range between 4 and 5.7, preferably 4.2 and 5.5 and in particular 4.5 and 5.3.

15. The use of a cosmetic cleaning agent according to one of claims 1 to 14 as a hair shampoo, bath and shower gel, hair conditioner, hair masque, aftershave and/or deodorant, preferably as a cleaning agent used for cleaning the hair and scalp.

## Revendications

1. Agent nettoyant cosmétique contenant dans un support cosmétiquement acceptable, sur la base de son poids total,
a) de 6 à 12% en poids d'au moins un tensioactif anionique,
b) de 0,01 à 1% en poids d'au moins un polymère de guar cationique,
c) de 0,05 à 2% en poids d'au moins une émulsion de silicone dans laquelle les particules de silicone ont un diamètre moyen de 600 nm maximum, et
d) de 0,02 à 1% en poids d'au moins une cire.

2. Agent nettoyant cosmétique selon la revendication 1, **caractérisé en ce qu'**il contient au moins un tensioactif anionique choisi parmi les sels d'alkylsulfate et/ou d'éthersulfates d'alkylpolyglycol de la formule R-(OCH₂-CH₂)ₓ-OSO₃ X dans laquelle R représente de préférence un groupe alkyle linéaire ou ramifié, saturé ou insaturé ayant de 8 à 30 atomes de carbone, x étant 0 ou un nombre de 1 à 12 et X représentant un ion de métal alcalin, un ion d'alcalino-terreux, un ion ammonium ou un ion alcanolamine.

3. Agent nettoyant cosmétique selon l'une des revendications 1 ou 2, **caractérisé en ce que** le polymère cationique b) est choisi parmi les dérivés hydroxy-alkyle en C₁ à C₄-guar cationique, de préférence l'hydroxyéthyltriméthylammonium-guar cationique et/ou l'hydroxypropyltriméthylammonium-guar cationique ayant des poids moléculaires moyens (moyenne pondérale) de 100000 à 2 000 000 Dalton, et des densités de charge cationique d'au moins 0,5 méq/g.

4. Agent nettoyant cosmétique selon l'une des revendications 1 à 3, **caractérisé en ce que** l'émulsion de silicone c) contient
(i) de 20 à 80% en poids, de préférence de 30 à 70% en poids et en particulier de 40 et 60% en poids d'au moins un polydialkylsiloxane, de préférence un polydiméthylsiloxane, et
(ii) au moins deux émulsifiants non ioniques différents qui sont choisis dans l'un des deux groupes constitués par les alcools en C₈ à C₂₄ 1 à 5 fois alcoxylés et les alcools en C₈ à C₂₄ 18 à 30 fois alcoxylés,
les quantités indiquées étant basées sur le poids de l'émulsion de silicone c).

5. Agent nettoyant cosmétique selon la revendication 4, **caractérisé en ce que** le polydiméthylsiloxane présente à 25°C une viscosité dans la gamme allant de 1 000 à 1 000 000 cSt, de préférence de 5 000 à 500 000 cSt, de façon particulièrement préférée de 10 000 à 200 000 cSt et en particulier de 30 000 à 100 000 cSt.

6. Agent nettoyant cosmétique selon l'une des revendications 1 à 5, **caractérisé en ce qu'**il contient une cire d) d'origine végétale qui a un point de fusion dans la gamme allant de 80 à 90°C.

7. Agent nettoyant cosmétique selon la revendication 6, **caractérisé en ce qu'**il contient une huile végétale hydrogénée, de préférence une huile de ricin hydrogénée.

8. Agent nettoyant cosmétique selon l'une des revendications 1 à 7, **caractérisé en ce qu'**il contient, sur la base de son poids total,
a) de 7 à 11% en poids et en particulier de 8 à 10% en poids d'au moins un tensio-actif anionique,
b) de 0,025 à 0,75% en poids et en particulier de 0,05 à 0,5% en poids d'au moins un des polymères de guar cationiques,
c) de 0,1 à 1,5% en poids et en particulier de 0,2 à 1% en poids d'au moins une émulsion de silicone qui a une taille de particules de silicone moyenne de 600 nm maximum, et
d) de 0,03 à 0,75% en poids et en particulier de 0,05 à 0,5% en poids d'au moins une cire.

9. Agent nettoyant cosmétique selon l'une des revendications 1 à 8, **caractérisé en ce qu'**il contient en outre, sur la base de son poids total, de 0,01 à 1% en poids, de préférence de 0,025 à 0,75% en poids et en particulier de 0,05 à 0,5% en poids d'au moins une huile végétale.

10. Agent nettoyant cosmétique selon l'une des revendications 1 à 9, **caractérisé en ce qu'**il contient, sur la base de son poids total,
a) 6 à 12% en poids, de préférence de 7 à 11% en poids et en particulier de 8 à 10% en poids d'au moins un alkyléthersulfate linéaire ou ramifié de la formule R-(OCH₂-CH₂)ₓ-OSO₃ X qui comporte un radical alkyle (R) ayant de 8 à 18 et en particulier de 10 à 16 atomes de carbone et de 1 à 6 et en particulier de 2 à 4 unités d'oxyde d'éthylène (X),
b) de 0,01 à 1%, de préférence de 0,025 à 0,75% en poids et en particulier de 0,05 à 0,5% d'au moins un des polymères de guar cationiques, connu sous le nom INCI chlorure d'hydroxypropyltrimonium de guar, qui a un poids moléculaire (moyenne pondérale) de 100 000 à 2 000 000 daltons, de préférence de 400 000 à 1 750 000 daltons et notamment de 800 000 à 1 600 000 daltons et une densité de charge cationique d'au moins 0,5 méq/g,
c) de 0,05 à 2% en poids, de préférence de 0,1 à 1,5% en poids et en particulier de 0,2 à 1% en poids d'au moins une émulsion de diméthylpolysiloxane ayant une taille de particule moyenne de silicone comportant de 600 nm maximum,
d) de 0,02 à 1% en poids, de préférence de 0,03 à 0,75% en poids et en particulier de 0,05 à 0,5% en poids d'au moins une huile végétale hydrogénée, de préférence de l'huile de ricin hydrogénée, et
e) de 0,01 à 1% en poids, de préférence de 0,025 à 0,75% en poids et en particulier de 0,05 à 0,5% en poids d'huile d'amande (douce), d'huile de noyau de pêche, d'huile de noyau d'abricot, d'huile d'avocat, d'huile de soja, d'huile de sésame, d'huile de tournesol , d'huile de pépins de raisin, d'huile de graines d'amarante, d'huile d'argan, d'huile d'olive, d'huile de jojoba et/ou de mélanges de ces huiles.

11. Agent nettoyant cosmétique selon l'une des revendications 1 à 10, **caractérisé en ce qu'**il contient en outre, sur la base du poids total, de 0,1 à 5% en poids, de préférence de 0,2 à 4% en poids et en particulier de 0,3 à 3% en poids, d'au moins un tensio-actif amphotère, zwittérionique et/ou non-ionique.

12. Agent nettoyant cosmétique selon la revendication 9, **caractérisé en ce qu'**il contient au moins un des tensio-actifs connus sous les noms INCI cocoamphoacétates de sodium, cocoamphodiacétates disodiques, cocoamphopropionates de sodium, cocoamphodipropionates disodiques, bétaïnes de coco, lauryl bétaïnes, cocamidopropyl bétaïne et/ou cocamides MEA.

13. Agent nettoyant cosmétique selon l'une des revendications 1 à 12, **caractérisé en ce qu'**il contient en outre, sur la base du poids total, de 0,025 à 7,5% en poids, de préférence de 0,05 à 5% et en particulier de 0,075 à 3% en poids d'au moins un principe actif antipelliculaire.

14. Agent nettoyant cosmétique selon l'une des revendications 1 à 13, **caractérisé en ce qu'**il a une valeur de pH dans la gamme de 4 à 5,7, de préférence de 4,2 à 5,5 et en particulier de 4,5 à 5,3.

15. Utilisation d'un agent nettoyant cosmétique selon l'une des revendications 1 à 14 comme shampooing, lotion de bain-douche, gel douche, shampooing, traitement capillaire, lotion de rasage et/ou déodorant, de préférence comme agent nettoyant qui est utilisé pour le nettoyage des cheveux et du cuir chevelu.
